# EUROPEAN PATENT APPLICATION

(11) **EP 2 236 158 A1**
(43) Date of publication of application: **06.10.2010**
(21) Application number: 09305265.2
(22) Date of filing: 30.03.2009
(51) Int. Cl.: A61K 45/06, A61K 31/4365, A61K 31/137, A61P 25/28

(54) **New therapeutic approaches for treating neuroinflammatory conditions**

(71) Applicant: Pharnext, 75014 Paris (FR)
(72) Inventor: Cohen,Daniel, 78110 Le Vesinet (FR); Nabirotchkin, Serguei, 92290 Chatenay Malabry (FR); Chumakov, Ilya, 77000 Vaux le Penil (FR)
(74) Representative: Becker, Philippe

(57) **Abstract**

The present invention discloses new compositions which can be used for the treatment of the neuroinflamation, in particular associated with neurodegenerative, autoimmune, infectious, toxic or traumatic disorders. More particularly, the invention relates to combined therapies for treating neuroinflammation by affecting extravasation cascade. The invention also discloses new methods for treating neuroinflammation pathological conditions in a subject.

## Description

### FIELD OF THE INVENTION

The present invention relates generally to the fields of pharmacology and medicine. The present invention discloses in particular new compositions which can be used for the treatment of the neuroinflammation, particularly associated with neurodegenerative, autoimmune, infectious, toxic or traumatic disorders. The invention also discloses new methods for treating neuroinflammation pathological conditions in a subject.

### BACKGROUND OF THE INVENTION

Neuroinflammation is a major hallmark of many neurological disorders (Block & Hong, 2005). Involvement of this process is well-known in neurological disorders, such as Multiple sclerosis (MS), Alzheimer's disease (AD), Parkinson's disease (PD), Amyotrophic lateral sclerosis (ALS), Acute disseminated encephalomyelitis (ADEM) and Neuromyelitis optica (NMO) described here only as the examples.

This condition of neuroinflammation mainly involves two types of immune cells: microglia (Stoll & Jander, 1999) and leukocytes (Man et al., 2007). Microglia are resident cells of the central nervous system (CNS) (Kreutzberg, 1996) and participate in its immune surveillance and defence. They are activated under pathological conditions and acquire functions that finally lead to degeneration processes by damaging or killing neurons (Tilleux & Hermans, 2007). Leukocytes are located throughout the body, including the blood and lymphatic system. Under physiological condition, only small numbers of leukocytes such as T lymphocytes are present in CNS parenchyma. Their passage is limited by the blood-brain-barrier (BBB) (Wekerle et al., 1986; Hickey et al., 1991; Carvey et al., 2005), which is a hermetic barrier made of endothelial cells that controls the access of blood stream elements to the CNS (Rubin & Staddon, 1999; Prat et al., 2001). Under pathological condition, hematogenous leukocytes readily leave blood stream and reach the parenchyma to participate to a destructive inflammatory response (Man et al., 2007; Cardona et al., 2008), since it has been shown that BBB integrity is impaired during inflammation (Lossinsky & Shivers, 2004). Extravasation and entry of leukocytes into the brain parenchyma follows a multistep paradigm that includes rolling, activation, adhesion, locomotion, protrusion, and transmigration steps (Man et al., 2007). This process requires interactions between leukocytes integrins and counter receptors of endothelial cells such as ICAM and VCAM (Man et al., 2007).

Precise pathogenesis of AD remains unclear. However, it has been hypothesized that AD is manifested by BBB impairment and neuroinflammation. Indeed, an increased number of Ig (Ishii & Haga, 1976; Mann et al., 1982; Licandro et al., 1983) has been found in the brain parenchyma, as well as CD4 or CD8 T cells (Itagaki et al., 1988; Rogers et al., 1988; McGeer et al., 1989; Singh, 1997; Neumann, 2001) in the hippocampus and temporal cortex of AD patients. Major histocompatibility complex (MHC) class I and II molecules, involved in antigen presentation and binding to T cells have also been identified in areas showing hallmark pathology (Itagaki et al., 1988; Rogers et al., 1988; McGeer et al., 1989; Mattiace et al., 1990; Perlmutter et al., 1992; Gonzalez-Scarano & Baltuch, 1999; Szpak et al., 2001; Kim & de Vellis, 2005; Walker & Lue, 2005). PD is another neurodegenerative disorder of unknown aetiology. BBB impairment has been hypothesized as a causative mechanism in PD (Kortekaas et al., 2005). It has been suggested that under inflammatory state, VCAM-1 and ICAM-1 receptors are upregulated, due to microglial release of proinflammatory cytokines (Neumann & Wekerle, 1998). This upregulation results in recruitment of T cells and monocytes that harbor the several adequate counter receptors (CD11a/CD18 (LFA-1) and very late antigen-4) (Neumann & Wekerle, 1998).

Acute disseminated encephalomyelitis (ADEM) is an immune-mediated inflammatory disorder of the CNS. It is a monophasic disease that can arise spontaneously. However, 5 to 25% of patients experience relapse (Marchioni et al., 2005; Tenembaum et al., 2002) with recurrent or multiphasic forms. The clinical course is fast and generally develops over hours to days (mean, 4.5 days). Most important symptoms include fever, headache, drowsiness, seizures and coma. Even if in the beginning the symptoms are generally mild, they get worse in a few days (Tenembaum et al., 2002). The mortality rate can reach 5 % (Menge et al., 2007). Syndromes of the peripheral nervous system (PNS) such as acute polyradiculoneuropathy may occur in ADEM but are rare (Amit et al., 1986; Amit et al., 1992; Nadkarni & Lisak, 1993).

The exact aetiology of ADEM is at present unknown. It is characterized by a widespread of demyelination in the white matter of the brain and spinal cord. It can also involve the cortex and deep gray matter structures. From a histological point of view, ADEM is characterized by perivenular infiltrates of T cells and macrophages, associated with demyelination (Tenembaum et al., 2007). Axonal damage has also been identified in the brains of some patients (DeLuca et al., 2004; Ghosh et al., 2004). It has been suggested that microbial infections that precede ADEM provoke a cross-reactive anti-myelin response through molecular mimicry. ADEM may also results of the activation of existing myelin-reactive T cell clones involved in a nonspecific inflammatory process (Tenembaum et al., 2007). ADEM is compared to multiple sclerosis, since it involves autoimmune demyelination (Rust, 2000; Poser, 2008).

Its incidence rate is 0.8 per 100,000 people per year (Leake et al., 2004). It affects more often children than adults, with a mean age at presentation that ranges from 5 to 8 years. ADEM is triggered either by infection or vaccination: around 70 to 77% of patients report a clinically evident antecedent infection or vaccination during the prior few weeks (Tenembaum et al., 2002; Amit et al., 1986; Hynson et al., 2001; Anlar et al., 2003). Nevertheless, many cases remain unexplained (Tenembaum et al., 2007). Genetic susceptibility was associated with the class II HLA-DRB (Idrissova Zh et al., 2003), HLA-DRB1 (Idrissova Zh et al., 2003; Oh et al., 2004), HLA-DRB5 (Oh et al., 2004) genes. Since no specific biologic markers exist, the diagnosis of ADEM is still based on the clinical and radiologic features.

No standard therapy exists for ADEM since results are generally obtained from case reports and small series. Treatments usually comprise nonspecific immunosuppressant therapy, such as steroids, immunoglobulin, or plasma exchange, which are used in other autoimmune diseases including MS (Tenembaum et al., 2007).

Neuromyelitis optica (NMO) is an infrequent autoimmune, inflammatory and demyelinating disease of the CNS that affects myelin of the neurons placed at the optic nerves and spinal cord. The disease can be either monophasic or relapsing (Ghezzi et al., 2004).

Extensive inflammation of the optic nerve (optic neuritis) and spinal cord (myelitis) usually leads to severe, permanent, relapse-related neurologic impairment (e.g., blindness, paraplegia) within 5 years (Wingerchuk & Weinshenker, 2003). Hallmarks of NMO are inflammatory lesions, cavitation, necrosis and axonal pathology. They have been observed in both grey and white matter of the spinal cord and optic nerves (Lucchinetti et al., 2002). At disease onset, the brain parenchyma is normal or may demonstrate few nonspecific subcortical white matter changes. It has been suggested that asymptomatic brain lesions are frequent in NMO at a later stage of the disease (Pittock et al., 2006a). Until recently, NMO was considered to be a variant of multiple sclerosis. However, clinical, neuroimaging, laboratory and pathological characteristics differ. For example, NMO attacks are not mediated by T cells but rather by B cells in an autoimmune manner (Lucchinetti et al., 2002), together with complement activation and NMO-immunoglobulin G antibodies that target aquaporin-4. This protein is the most abundant water channel located in the foot processes of the astrocytes that surround BBB (Nielsen et al., 1997; Pittock et al., 2006b). Thus NMO is at present distinguished from MS (Wingerchuk et al., 1999).

NMO represent less than 1% of demyelinating diseases of the CNS in Caucasians. Percentage is increased in non-Caucasians, certainly more common in Asians. NMO is more common in women (2/3 of patients) and they experience a more severe course (Ghezzi et al., 2004). The median age at onset is in the late 30s, rather than MS that usually begins in the late 20s (Wingerchuk & Weinshenker, 2003). There are few reports of familial cases of NMO and the majority of patients have no affected relatives (Wingerchuk et al., 1999). Genetic susceptibility was associated with the class II HLA-DPB1 gene in Japanese patients with optic-spinal MS (OSMS), a variant form of NMO developed in tropical and subtropical regions (Cree et al., 2004). NMO has sporadically been associated with systemic diseases such as collagen vascular diseases, auto-antibody syndromes, with infections with varicella-zoster virus, Epstein-Barr virus, HIV, and with exposure to clioquinol and antituberculosis drugs (Cree et al., 2002).

There is at the moment no established optimal treatment for NMO since no randomized controlled trials have been performed. At present, parenteral corticosteroids are widely employed as first-line treatment of optic neuritis and myelitis attacks (Mandler et al., 1998), whereas therapeutic plasmapheresis that aims at removing autoantibodies, immune complexes and inflammatory mediators from the plasma, is applied in the case of corticosteroids failure (Keegan et al., 2002; Lehmann et al., 2006). Various strategies for the prevention of NMO relapses have been employed in small case series with modest activity: combination of azathioprine with oral prednisone (Mandler et al., 1998); low-dose corticosteroid monotherapy (Wingerchuk & Weinshenker, 2005; Watanabe et al., 2007); standard immunomodulatory therapies (beta-interferons and glatiramer acetate) (Bergamaschi et al., 2003; Saida et al., 2005; Papeix et al., 2007; Warabi et al., 2007; Gartzen et al., 2007); immunosuppressive therapies such as mitoxantrone hydrochloride (Weinstock-Guttman et al., 2006); Rituximab (Weinstock-Guttman et al., 2006; Cree et al., 2005).

Multiple sclerosis (MS) is the most frequent non traumatic neurological disease among young adults in North America and Europe, with 3.6 and 2.0 cases per 100 000 person-years incidence for women and men respectively (Alonso & Heman, 2008). PPMS occurs with an equal incidence in male and female. Multiple factors such as genetics, environment and infectious agents are also part of MS development. It is considered as a non-herited disease. However, one can inherit a greater susceptibility to acquiring MS. For example, risk is increased when living in North America or Europe, with 0,1%. It reaches 2 to 3% for family members (Rosati, 2001). Increased risk of developing MS has also been associated with the major histocompatibility complex (MHC) class II (Trapp & Nave, 2008), including the HLA-DRB1 gene which accounts for 16 to 60% of the genetic susceptibility (Haines et al., 1998). This supports the involvement of immune system in MS physiopathology. Additional susceptibility genes have been associated with MS, such as Interleukin-7 and -2 receptor alpha chain that display a low odds ratio of 1.3 (Olsson & Hillert, 2008). It has been raised that MS is a complex disease involving multiple genes with a low penetrance (Olsson & Hillert, 2008). Indeed, it has been shown that experimental MS models are regulated by many genes with small effects, more than about 100. Studies in humans suggest a similar situation (Olsson & Hillert, 2008). Environmental factors have been associated with higher MS prevalence, such as sunlight exposure (Hayes, 2000; van Etten & Mathieu, 2005; Ponsonby et al., 2005), socioeconomic development, vitamin D status, smoking habits, and viral and microbial infections such as Epstein-Barr virus, varicella-zoster virus (Reviewed in (Pugliatti et al., 2008), (Sotelo, 2007; Kampman & Brustad, 2008; Lincoln et al., 2008; Posnett, 2008).

Multiple sclerosis is considered as an inflammatory demyelinating disease of the CNS (Skaper, 2007; Lassmann et al., 2007). For 85% of patients, disease course begins with a phase of recurrent and reversible neurological troubles termed Relapsing-Remitting MS (RRMS). This condition appears by the third-fourth decade of life. It can last for years and decades, with alternate phases of attacks with relapses, during which the patients recuperate neurological function (Trapp & Nave, 2008). Attacks last from a few days to weeks, and remissions a few months to years. After 8 to 20 years, patients enter the Secondary Progressive MS (SPMS) that is characterized by a continuous and irreversible neurological decline. A rarer disease form named Primary Progressive MS (PPMS) affects 15% of MS patients. There are no relapses occurring in PPMS disease form and disease is progressive from the onset. It occurs later than RRMS form (39 vs 29 years). Fifty percent of MS patients are unable to perform household and employment responsibilities 10 years after disease onset, and 50% are nonambulatory 25 years after disease onset (Trapp & Nave, 2008). Morphological alterations in CNS anatomy lead to paralysis, sensory disturbances, lack of coordination, and visual impairment among the most common features. These alterations (detected by magnetic resonance imaging (MRI), histopathologic evaluations and disease course vary significantly among patients (Agrawal & Yong, 2007).

Inflammation, breakdown of BBB, demyelination, and axonal transection are pathological features of acute MS lesions. In RRMS phase, disability is caused by focal areas of inflammation where myelin, oligodendrocytes (responsible of myelin formation) and axons are destroyed (Ganter et al., 1999; Bjartmar et al., 2000; Lovas et al., 2000; Trapp et al., 1998). Attention is primarily focused on demyelinated lesions in the white matter at the chronic stage of the disease. However, evidence has accumulated that large areas of grey matter are also affected in MS patients (Stadelmann et al., 2008). It has been shown that T cells (mainly MHC-class I restricted CD8+ T cells) participate actively to inflammation, in addition to activated microglia (Lassmann et al., 2007). Moreover, impairment of BBB has been observed (Hochmeister et al., 2006; Kirk et al., 2003), allowing T cells to enter CNS. Relapse lasts a few month and the patients recuperate neurological function, due to resolution of inflammation and remyelination (Trapp et al., 1998; Ferguson et al., 1997). Transition toward SPMS and PPMS stages occurs when CNS can no longer compensate for additional neuronal loss (Trapp et al., 1999). In SPMS and PPMS stages, focal demyelinated white matter lesions remain, but new inflammatory active demyelinating lesions are infrequent. The pre-existing active lesions expand slowly, showing a little myelin breakdown activity especially in margins. These lesions show moderate inflammatory infiltrates, principally composed of T cells (CD8+ T cells) and active microglia (Prineas et al., 2001). In addition, diffuse atrophy of the grey and white matter as well as 'normal-appearing white matter' (NAWM) are observed (Miller et al., 2002). NAWM is characterized by a diffuse inflammatory process and generalized activation of microglia, associated with axonal damage and secondary demyelination (Allen and McKeown, 1979; Allen et al., 2001; Kutzelnigg et al., 2005). Cortical damages are additional characteristics of the progressive stage.

Disease mechanisms pertinent to neuroinflammation have often been inferred from the Experimental Autoimmune Encephalomyelitis (EAE), an animal model of MS. This model is induced by sensitization of animals with brain tissue, myelin or protein antigens or by passive transfer of autoreactive T cells (Lassmann, 2008). Animals develop an inflammatory demyelinating disease that closely looks like MS (Lassmann, 2008). These models support the hypothesis according to which MS is an autoimmune disease. Indeed immunological data show autoreactive T cells and autoantibodies in circulation and in the cerebrospinal fluid. However, it is presumed that MS pathogenesis is a bit different than predicated from EAE studies (McFarland & Martin, 2007), since divergent results regarding therapeutic response were observed. This divergence could be explained by the difference in inflammatory responses cells. Indeed, inflammation in EAE is mediated by MHC-type II cells, mainly CD4+ T lymphocytes, at least at early stages of lesion development (Flugel et al., 2001). Since inflammation is a main hallmark of acute MS lesions, aggressive anti-inflammatory strategies have been assessed during RRMS, performing neuroprotective effects. Interferon β (IFNβ) and glatiramer acetate (GA) are commonly used to treat RRMS. IFNβ inflammatory effects concern decrease of antigen presentation, apoptosis, and entry of immune cells into the CNS (Neuhaus et al., 2005). GA mimics myelin basic protein (MBP), a major component of CNS myelin. It reduces antigen presentation and stimulates T cell secretion of cytokines associated with anti-inflammatory or lymphocytes T helper 2 actions (Neuhaus et al., 2001). Natalizumab is a humanized monoclonal antibody specific for α4 integrins. It suppresses the binding of leukocytes to vascular endothelia, thus targeting entry of immune cell in the CNS (Yednock et al., 1992). It reduced new enhancing MS lesions by 90% for the treatment of RRMS in phase II studies (Polman et al., 2006; O'Connor et al., 2004). However, a rare and often fatal virus-induced demyelinating disease (progressive multifocal leukoencephalopathy) occurred in 3 patients. It is now used for aggressive disease courses that have failed INFβ and GA (Trapp & Nave, 2008). Recombinant erythropoietin was recently proposed for treating MS animal models (Li et al., 2004) and MS patients (Konstantinopoulos et al., 2007).

However, there are at present no therapies approved for PPMS or SPMS patients.

Consequently, there is a need for a treatment method for neuroinflammation conditions, particularly associated with neurodegenerative, immune, infectious, toxic or traumatic disorders, which are safe, efficient and well tolerated by patients.

### SUMMARY OF THE INVENTION

The purpose of the present invention is to provide new therapeutic approaches for treating neuroinflammatory condition, in particular specific for neurological diseases. The invention also relates to compositions and methods for treating neuroinflamation associated with neurodegenerative disorders by modulation or reversion of such condition in a subject. More specifically, an object of this invention relates to a composition comprising a combination of at least two compounds chosen from irbesartan, idraparinux or otamixaban, SR48692, mecamylamine and clopidogrel, or salts or prodrugs thereof. The invention relates also to a composition comprising a combination of at least two compounds chosen from the group consisting of a modulator of endothelium permeability, a modulator of extracellular matrix formation, a modulator of cell adhesion and motility, a modulator of shear stress on endothelium and a modulator of platelets aggregation, or salts or prodrugs thereof. Preferably, the compositions according to the invention are used for treating neuroinflammation, particularly associated with neurodegenerative, autoimmune, infectious, toxic or traumatic disorders.

### DETAILED DESCRIPTION OF THE INVENTION

In the present invention, the inventors propose compounds or combinations of compounds, affecting specific stages of leukocyte transmigration, which can be used for treatment of neurodegenerative, autoimmune, infectious, toxic or traumatic disorders, where inflammatory component could be suggested as aetiological or pathology-exacerbating factor. The inventors have also identified several compounds which, in combination(s) or alone, can effectively affect such pathways leading to neuroinflammation, and represent new therapy for the treatment of these disorders.

The invention therefore provides novel compositions, which can be used for treating neuroinflammation.

In a preferred embodiment, the composition according to the invention comprises a combination of at least two compounds chosen from the group consisting of irbesartan, idraparinux, otamixaban, SR48692, mecamylamine, clopidogrel, or salts or prodrugs or derivatives or sustained release formulations thereof for simultaneous, separate or sequential administration.

In a particular embodiment, the composition according to the invention comprises a combination of at least two compounds chosen from the group consisting of irbesartan, idraparinux, otamixaban and SR48692, salts, prodrugs, derivatives or sustained release formulations thereof.

In a further embodiment, the composition according to the invention comprises a combination of at least irbesartan, idraparinux, otamixaban and SR48692, salts, prodrugs, derivatives or sustained release formulations thereof.

In a further embodiment, the composition according to the invention comprises a combination of at least irbesartan, idraparinux, otamixaban, mecamylamine and SR48692, salts, prodrugs, derivatives or sustained release formulations thereof.

In a further particular embodiment, the composition according to the invention comprises a combination of at least mecamylamine and clopidogrel, or salts, prodrugs, derivatives or sustained release formulations thereof.

In a preferred embodiment, the above compositions according to the invention further comprise one or more pharmaceutically acceptable excipients.

In another embodiment, the composition of the invention comprises a combination of at least two compounds chosen from the group consisting of a modulator of endothelium permeability, a modulator of extracellular matrix formation, a modulator of cell adhesion and motility, a modulator of shear stress on endothelium, a modulator of platelets aggregation, or salts or prodrugs or derivatives or sustained release formulations thereof.

In another embodiment, the composition according to the invention comprises a combination of at least two compounds chosen from the group consisting of a modulator of endothelium permeability, a modulator of extracellular matrix formation, a modulator of cell adhesion and motility, a modulator of shear stress on endothelium and a modulator of platelets aggregation, or salts or prodrugs thereof, and one or more pharmaceutically acceptable excipients.

In another preferred embodiment, the composition according to the invention comprises a combination of at least two compounds chosen from the group consisting of irbesartan, idraparinux, otamixaban, SR48692, mecamylamine, clopidogrel, or salts or prodrugs or derivatives or sustained release formulations thereof, for treating neuroinflammation, particularly associated with neurodegenerative, autoimmune, infectious, toxic or traumatic disorders.

In another embodiment, the composition according to the invention comprises a combination of at least two compounds chosen from a group consisting of a modulator of endothelium permeability, a modulator of extracellular matrix formation, a modulator of cell adhesion and motility and a modulator of platelets aggregation, or salts or prodrugs or derivatives or sustained release formulations thereof, for treating neuroinflammation, particularly associated with neurodegenerative, autoimmune, infectious, toxic or traumatic disorders.

Preferably, said neurodegenerative disorder is selected from the group consisting of but limiting to multiple sclerosis (MS), Alzheimer's disease (AD), Parkinson's disease (PD), Amyotrophic lateral sclerosis (ALS), Acute disseminated encephalomyelitis (ADEM) and Neuromyelitis optica (NMO).

A particular object of this invention is a composition comprising a combination of at least two compounds chosen from irbesartan, idraparinux, otamixaban, SR48692, mecamylamine and clopidogrel, or salts or prodrugs thereof or derivatives or sustained release formulations, for treating multiple sclerosis (MS).

In a particular embodiment, a composition according to the invention comprises compounds that modify leukocyte extravasation cascade by modulating endothelium permeability, extracellular matrix formation, cell adhesion and motility, shear stress on endothelium and platelets aggregation.

The present invention relates also to a composition comprising at least a modulator of endothelium permeability (preferably selected from bosentan, fondaparinux, pentazocine, nifuroxazide, tiludronate, gliclazide, irbesartan, loperamide), a modulator of extracellular matrix formation (preferably selected from argatroban, lisinopril, quinapril, ramipril, idraparinux, otamixaban, enoxaparin, disulfiram, spironolactone), a modulator of cell adhesion and motility (preferably selected from terbinafine, lithium carbonate, valproic acid, diosmin, captopril, metformin, cromoglicate, bacitracin, eflornithine, benzbromarone, SR48692, glibenclamide, tranexamic acid), a modulator of shear stress on endothelium (preferably selected from amlodipine, clonidine, liothyronine, diltiazem, gentamicin, mecamylamine, neomycin, streptomycin) and a modulator of platelets aggregation (preferably selected from cilostazol, tirofiban, clopidogrel, ticlopidine).

In other embodiments, the composition of the invention comprises at least one of the following combinations of compounds, for combined, separate or sequential administration:
- at least one modulator of endothelium permeability (preferably selected from bosentan, fondaparinux, pentazocine, nifuroxazide, tiludronate, gliclazide, irbesartan, loperamide) and at least one modulator of platelets aggregation (preferably selected from cilostazol, tirofiban, clopidogrel, ticlopidine);
- at least one modulator of extracellular matrix formation (argatroban, lisinopril, quinapril, ramipril, idraparinux, otamixaban, enoxaparin, disulfiram, spironolactone) and least one modulator of platelets aggregation (preferably selected from cilostazol, tirofiban, clopidogrel, ticlopidine);
- at least one modulator of cell adhesion and motility (preferably selected from terbinafine, lithium carbonate, valproic acid, diosmin, captopril, metformin, cromoglicate, bacitracin, eflornithine, benzbromarone, SR48692, glibenclamide, tranexamic acid) and at least one modulator of platelets aggregation (preferably selected from cilostazol, tirofiban, clopidogrel, ticlopidine);
- at least one modulator of shear stress on endothelium (preferably selected from amlodipine, clonidine, liothyronine, diltiazem, gentamicin, mecamylamine, neomycin, streptomycin) and at least one modulator of platelets aggregation (preferably selected from cilostazol, tirofiban clopidogrel, ticlopidine);
- at least one modulator of extracellular matrix formation (argatroban, lisinopril, quinapril, ramipril, idraparinux, otamixaban, enoxaparin, disulfiram, spironolactone) and at least one modulator of endothelium permeability (preferably selected from bosentan, fondaparinux, pentazocine, nifuroxazide, tiludronate, gliclazide, irbesartan, loperamide);
- at least one modulator of extracellular matrix formation (argatroban, lisinopril, quinapril, ramipril, idraparinux, otamixaban, enoxaparin, disulfiram, spironolactone) and at least one modulator of cell adhesion and motility (preferably selected from terbinafine, lithium carbonate, valproic acid, diosmin, captopril, metformin, cromoglicate, bacitracin, eflornithine, benzbromarone, SR48692, glibenclamide, tranexamic acid);
- at least one modulator of cell adhesion and motility (preferably selected from terbinafine, lithium carbonate, valproic acid, diosmin, captopril, metformin, cromoglicate, bacitracin, eflornithine, benzbromarone, SR48692, glibenclamide, tranexamic acid) and at least one modulator of endothelium permeability (preferably selected from bosentan, fondaparinux, pentazocine, nifuroxazide, tiludronate, gliclazide, irbesartan, loperamide);
- at least one modulator of cell adhesion and motility (preferably selected from terbinafine, lithium carbonate, valproic acid, diosmin, captopril, metformin, cromoglicate, bacitracin, eflornithine, benzbromarone, SR48692, glibenclamide, tranexamic acid) and at least one modulator of shear stress on endothelium (preferably selected from amlodipine, clonidine, liothyronine, diltiazem, gentamicin, mecamylamine, neomycin, streptomycin);
- at least one modulator of endothelium permeability (preferably selected from bosentan, fondaparinux, pentazocine, nifuroxazide, tiludronate, gliclazide, irbesartan, loperamide) and at least one modulator of shear stress on endothelium (preferably selected from amlodipine, clonidine, liothyronine, diltiazem, gentamicin, mecamylamine, neomycin, streptomycin).

The invention also relates to a composition comprising at least one modulator of extracellular matrix formation, preferably idraparinux or otamixaban, and at least one modulator of platelets aggregation, preferably clopidogrel, ticlopidine or tirofiban.

In another embodiment, said composition comprises at least one modulator of cell adhesion and motility, preferably, terbinafine, and at least one modulator of platelets aggregation, preferably clopidogrel, ticlopidine or tirofiban.

In another embodiment, said composition comprises at least one modulator of extracellular matrix formation, preferably idraparinux or otamixaban, and at least one modulator of cell adhesion and motility, preferably tranexamic acid.

In another embodiment, said composition comprises at least one modulator of extracellular matrix formation, preferably idraparinux or otamixaban, and at least one modulator of cell adhesion and motility, preferably neurotensin receptor antagonist SR48692.

In another embodiment, said composition comprises at least one modulator of cell adhesion and motility, preferably, bacitracin, and at least one modulator of platelets aggregation, preferably clopidogrel, ticlopidine or tirofiban.

In another embodiment, said composition comprises at least one modulator of cell adhesion and motility, preferably diosmin, and at least one modulator of platelets aggregation, preferably clopidogrel, ticlopidine or tirofiban.

In another embodiment, said composition comprises at least one modulator of endothelium permeability, preferably, irbesartan, and at least one modulator of platelets aggregation, preferably clopidogrel, ticlopidine or tirofiban.

In another embodiment, said composition comprises at least one modulator of cell adhesion and motility, preferably tranexamic acid, and at least one modulator of platelets aggregation, preferably clopidogrel, ticlopidine or tirofiban.

In another embodiment, said composition comprises at least one modulator of cell adhesion and motility, preferably neurotensin receptor antagonist SR48692, and at least one modulator of platelets aggregation, preferably, clopidogrel, ticlopidine or tirofiban.

In another embodiment, said composition comprises at least one modulator of extracellular matrix formation, preferably argatroban, and at least one modulator of cell adhesion and motility, preferably tranexamic acid.

In another embodiment, said composition comprises at least one modulator of shear stress on endothelium, preferably mecamylamine or neomycin, and at least one modulator of platelets aggregation, preferably clopidogrel, ticlopidine or tirofiban.

In another embodiment, said composition comprises at least a modulator of endothelium permeability, preferably irbesartan, a modulator of extracellular matrix formation, preferably idraparinux or otamixaban, a modulator of cell adhesion and motility, preferably SR48692, a modulator of shear stress on endothelium, preferably, mecamylamine, and a modulator of platelets aggregation, preferably clopidogrel.

In other particular embodiments, all the compositions according to the invention can be used for treating neuroinflammation, particularly in subjects having neurodegenerative disorders.

In other embodiments, the compounds of compositions according to the present invention can be administered simultaneously, separately or sequentially in a same subject. In other embodiments, said compounds are formulated in solid or liquid dosage forms, for single or repeated administration to a patient.

Preferably, the compositions of the invention further comprise one or more pharmaceutically acceptable excipients.

Another object of the invention resides in a therapeutic regimen which comprises a combination of at least two compounds chosen from the group consisting of a modulator of endothelium permeability, a modulator of extracellular matrix formation, a modulator of cell adhesion and motility, a modulator of shear stress on endothelium and a modulator of platelets aggregation, or salts or prodrugs thereof, and one or more pharmaceutically acceptable excipients, for simultaneous, separate or sequential administration.

The invention further provides a method for treating neuroinflammation, comprising administering to a subject in need thereof an effective amount of any compound or combination of compounds or compositions as disclosed above. A preferred method comprises the administration of a combination of at least two compounds selected from compounds or salts or prodrugs or derivatives or sustained release formulations thereof.

Any of the various uses or methods of treatment disclosed herein can also include an optional step of diagnosing a patient as having neuroinflammation, or identifying an individual as at risk of developing neuroinflammation.

A further object of this invention is a method for treating neuroinflammation in a subject, comprising administering simultaneously, separately or sequentially to said subject, in need of such a treatment, an effective amount of any combination of at least two compounds of compositions mentioned above, or salts or prodrugs thereof.

Another object of the invention resides in a method for treating neuroinflammation in a human or animal subject, said method comprising administering simultaneously, separately or sequentially by any oral, or parenteral or intrathecal or topical route or by inhalation or percutaneous or mucosal route to said subject, in need thereof, an effective amount of any combination of at least two compounds according to the invention, or salts or prodrugs or derivatives or sustained release formulations thereof.

In this regard, a further object of this invention is a method of treating neuroinflammation, the method comprising (1) assessing whether a subject has neuroinflammatory disesase and (2) treating the subject having this disease with an effective amount of a of a composition according to the invention. Determining whether a subject has neuroinflammation can be done by various tests known per se in the art, such as DNA assays.

Another particular object of the invention resides in a method for assessing neuroinflammation treatment efficacy in a subject, wherein cells derived from the subject are exposed in vitro to a composition of the invention.

The invention may be used for treating neuroinflammation in any mammalian subject, particularly human subjects. The present invention provides new therapeutic approaches for treating neuroinflammation condition characteristic for several neurological diseases. The invention discloses novel use of drug combinations which allow an effective correction of such diseases and may be used in any mammalian subject.

The invention describes drug combinations that correct migration of immune cells in CNS through blood-brain-barrier: a key step in pathogenesis of neuronal disorders that comprise an important inflammatory part.

Interaction of immune cells with endothelium compartment is a complex stepwise process strictly controlled at cellular and molecular levels. Transendothelial migration of immune cells into damaged tissues (extravasation) is a key physiological event implicated both in innate and adaptive immunity. Cooperative interaction of several cell types, namely endothelial cells, immune cells, platelets, vascular smooth muscle cells and pericytes, modulates passage of leukocytes to inflammation sites and assures tight functional control of immune responses.

In the present invention, the inventors have identified the combination of drugs that target the extravasation pathological pathway and modify it to improve the condition of patients suffering of neurodegenerative diseases. The different components of this pathway, associated in particular with remodelling of endothelium permeability, cell adhesion and motility, extracellular matrix formation, shear stress conditions and platelets aggregation, are providing different classes of drugs for combination treatments according to the invention.

Endothelial cells undergo profound functional remodelling in context of innate and adaptive acute or chronic immunity, facilitating different phases of inflammatory responses. Endothelial cells are primed by inflammatory stimuli to express broad spectrum of adhesive and chemoattractant molecules implicated in recruitment of leukocytes, their capture on vascular bed (cell adhesion) and trafficking into target tissues through activated endothelium (Jordan&Sessa, 2007; Vestweber, 2007).

### Changes in cell adhesive pathways of endothelial cells provoked by inflammatory stimuli

Inflammatory stimuli, such as histamine or cytokines TNFα or IL-1, provoke profound functional remodelling of endothelial cells and transform them to effective mediators of recruitment and passage of leukocytes to damaged tissues. For instance, pro-inflammatory cytokines suppress synthesis of NOS3 and thrombomodulin and stimulates production of tissue factor in activated endothelial cells, thereby decreasing their anti-coagulant properties (Bevilacqua et al., 1986; Sohn et al., 2005). Elevation of intracellular Ca2+ in activated endothelial cells stimulates exocytosis of Weibel-Palade bodies and exposure of adhesive molecule P-selectin on the surface of endothelium (Birch et al., 1992; Birch et al., 1994). In parallel, activated endothelial cells start to produce platelet-activating factor, chemokine IL-8 and E-selectin, which synergistically trigger tethering of leukocytes to endothelium and accelerate their transmigration (diapedesis) into inflamed tissues (Pan et al., 1998). Activated endothelial cells are also able to capture chemokines, produced by other cells, on their surface and thereby, create optimal microenvironment for leukocyte adhesion on primed endothelium.

### Role of endothelium permeability in inflammation process

Extravasation of leukocytes from vascular network could be subdivided into several morphologically and functionally distinct consecutive steps: leukocytes capture on endothelial cells, rolling of leukocytes on vascular bad, slow rolling preceding arrest of leukocytes, adhesion strengthening, spreading of leukocytes, intravascular crawling, transcellular or paracellular (junctional) transmigration and migration through the basement membrane.

### Role of cell adhesion pathways and shear stress conditions in interaction of leukocytes with endothelium

Specific adhesion of leukocytes to endothelium includes selectin-dependent transient capture and rolling of leukocytes on endothelium, followed by chemokine-triggered and integrin-mediated leukocyte arrest (Ley et al., 2007).

Tethering of immune cells by endothelium is initiated by cell adhesion molecules selectins, which mediate, so called, primary, transient interaction of leukocytes with endothelial cells (Butcher, 1991; Springer, 1994).

### Leukocyte arrest on endothelium is accompanied by remodelling of intracellular pathways, implicated in cell adhesion and motility

Transition from slow rolling stage to leukocytes arrest on endothelium is mediated by leukocyte integrins, which bind to their ligands ICAM1/2 and VCAM1, expressed on endothelial cells (Campbell et al., 1996; Campbell et al., 1998). Activation of integrins via specific GPCRs, is triggered by chemokines exposed on the surface of inflamed endothelium. Not only activated endothelial cells, but platelets and mast cells also participate in synthesis and secretion of chemokines and chemoattractants on the luminal surface of vascular walls.

Arrest of rolling leukocytes of vascular endothelium is followed by adhesion strengthening. Though molecular mechanisms underlying strengthening of leukocyte adhesion to inflamed endothelium are only started to emerge, the "outside-in" signalling by activated integrins is obviously important at this stage.

### Transmigration of leukocytes through endothelium depends on cooperative interaction between cellular pathways mediated endothelium permeability, cell adhesion and motility

The strong and specific adherence of leukocytes to endothelium, stimulated by inflammatory stimuli, is finalized by migration (diapedesis) of immune cells through the layer of endothelial cells and basement membrane into extravascular damaged tissues.

Leukocyte migration occurs either through cellular contacts in endothelium (junctional pathway) or through cytoplasm of endothelial cells (transcelluar pathway) (Kvietys & Sandig, 2001; Muller, 2001; Muller, 2003; Engelhardt & Wolburg, 2004).

### Cell adhesion pathways modulate endothelium permeability during leukocyte transmigration

Subsequent extension of leukocyte protrusions into endothelium depends on cooperative interactions of several distinct cell surface molecules, which orchestrate complex cytoskeleton reorganisation in both endothelial and immune cells. For instance, engagement of endothelial ICAM1 ligand by its cognate integrin receptors on migrating leukocytes activates p38 kinase and RHO GTPase in endothelial cells, which could cooperatively, via myosin light-chain kinase/VE-cadherin and via PLCγ1/PKC/Src/cortactin pathways, enhance contraction of endothelial cells and increase opening of intercellular contacts (Durieu-Trautmann et al., 1994; Etienne et al., 1998; Adamson et al., 1999; Tilghman & Hoover, 2002; Greenwood et al., 2003; Millan & Ridley, 2005).

Besides ICAM-1 and VCAM-1, several other molecules were disclosed as important positive modulators of leukocyte transmigration. For instance, experimental disruption of the transmembrane PECAM-1 (CD31) protein, implicated in homophilic intercellular interactions in endothelium, reduces dramatically neutrophil extravasation in vivo (Schenkel et al., 2004a; Schenkel et al., 2004b).

Furthermore, two other proteins could potentially interact with PECAM-1 protein in leukocyte transmigration, human poliovirus receptor PVR protein, modifier of LFA-1 function related to nectins, and a homophilic leukocyte adhesion molecule CD99, which could be also detected on the surface of endothelial cells.

### VE-mediated pathways controlling endothelium permeability as a main negative regulator of leukocyte transmigration

Contrarily to previously described modulators of leukocyte extravasation, VE-cadherin is a main negative regulator of leukocyte transmigration at adherent junctions of endothelial cells: passage of leukocytes via junctional pathway requires displacement of VE-cadherin from endothelial cell contacts. VE-cadherin, expressed at adherens junctions of endothelial cells, is associated with α-, β-and γ-catenins, which mediate its interaction with the actin cytoskeleton. The endothelial-specific phosphatase VE-PTP forms complex with VE-cadherin, and probably, represents natural functional regulator of VE-cadherin-dependent adhesive properties in endothelium, since knock-down of VE-PTP phosphatase was demonstrated to potentiate transendothelial migration of neutrophils (Nawroth et al., 2002; Vestweber, 2007).

### Role of extracelluar matrix in leukocyte migration through endothelium

At the last steps of leukocyte extravastion from vascular system, immune cells need to penetrate endothelial basement membrane and sheath of pericytes. Migration of leukocyte through basement membrane, as their migration through endothelial cells, is accompanied by profound adaptive functional remodelling of invading immune cells. For instance, migrating leukocytes activates the β1-, β2- and β3-integrin families and mobilizes α6β1-integrin, the main leukocyte receptor for laminin, from intracellular stores to the cell surface, thereby potentiating leukocyte interaction with extracellular proteins of basement membrane (Dangerfield et al., 2002; Newman & Newman, 2003).

Another important class of proteins, mobilized by migrating immune cells and cooperating with integrins in extravascular migration, are represented by cell-surface leukocyte proteases, which facilitate interaction of leukocyte receptors with extracellular ligands or generate chemotactic gradients inside extracellular matrix (Adair-Kirk et al., 2003).

### Platelets as cellular regulators of extravasation cascade

Though leukocytes and endothelial cells are major players in extravasation cascade, passage of immune cells to damage tissues could be significantly influenced by other cell types.

Platelets could be recognized as one of the most powerful modulator of leukocyte interaction with activated endothelium; they are tightly involved in control of blood homeostasis and contribute significantly to activation of coagulation cascade and thrombus formation, provoked by endothelial damage.

Reciprocally, platelets regulate activity of injured/inflamed endothelium. Platelets cover injured vessel area, and thereby serve as a mechanical substrate for leukocyte tethering.

As has been reviewed above, migration of leukocytes through endothelium is assured by tight cooperation between immune and endothelial cells, and is accompanied by their functional remodelling, which is manifested by changes in endothelium permeability, by activation of cell adhesion and motility pathways in endothelial and immune cells, and by adaptive modifications of shear stress conditions and extracellular matrix formation in vascular network. Primed by inflammatory stimuli, endothelial cells express cell adhesion molecules, which, upon successful ligation by their cognate leukocytes receptors, induce changes in endothelium permeability facilitating passage of tethered leukocytes. Complex interaction of cell surface molecules on immune and endothelial cells also provide guiding signals, which assure coordinated activation of signalling pathways modulating leukocyte cell motility. Adaptive changes in shear stress conditions and extracellular matrix formation create optimal microenvironment for productive interaction between endothelial and immune cells and successful leukocyte migration into inflamed tissues. Finally, passage of leukocytes through endothelium could be modulated by numerous external stimuli, provided by other cell types, namely by the activated platelets, which are considered as multifunctional regulators of extravasation cascade.

Therefore, the inventors have identified compounds able to modify principal functional processes, implicated in leukocyte extravasation, namely: remodelling of endothelium permeability, cell adhesion and motility, extracellular matrix formation, shear stress conditions and platelets aggregation, as promising candidates for combinatorial therapeutic intervention in context of human neuroinflammatory diseases.

Taking into account functional complexity of extravasation cascade, the inventors consider combinatorial treatment as the most appropriate approach for effective therapeutic modulation of inflammatory conditions.

Compounds of the invention that modify leukocyte extravasation cascade include:
- modulators of **remodeling of endothelium permeability**, preferably selected from bosentan (CAS number 147536-97-8), fondaparinux (CAS number 114870-03-0), pentazocine (CAS number 359-83-1), nifuroxazide (CAS number 965-52-6), tiludronate (CAS number 89987-06-4), gliclazide (CAS number 21187-98-4), irbesartan (CAS number 138402-11-6), loperamide (CAS number 53179-11-6),
- modulators of **cell adhesion and motility** preferably selected from terbinafine (CAS number 91161-71-6), lithium carbonate (CAS number 554-13-2), valproic acid (CAS number 99-66-1), diosmin (CAS number 520-27-4), captopril (CAS number 62571-86-2), metformin (CAS number 657-24-9), ketoprofen (CAS number 22071-15-4), cromoglicate (CAS number 16110-51-3), bacitracin (CAS number 1405-87-4), eflornithine (CAS number 67037-37-0), benzbromarone (CAS number 3562-84-3), SR48692 (IUPAC Name: 2-[[1-(7-chloroquinolin-4-yl)-5-(2,6-dimethoxyphenyl) pyrazole-3-carbonyl]amino]adamantane-2-carboxylic acid), glibenclamide (CAS number 10238-21-8), tranexamic acid (CAS number 1197-18-8),
- modulators of **extracellular matrix formation** preferably selected from argatroban (CAS number 74863-84-6), lisinopril (CAS number 83915-83-7), quinapril (CAS number 85441-61-8), ramipril (CAS number 87333-19-5), idraparinux (CAS number 162610-17-5), otamixaban (CAS number 193153-04-7), enoxaparin (CAS number 9005-49-6), disulfiram (CAS number 97-77-8), spironolactone (CAS number 52-01-7),
- modulators of **shear stress on endothelium** preferably selected from amlodipine (CAS number 88150-42-9), clonidine (CAS number 4205-90-7), liothyronine (CAS number 6893-02-3), diltiazem (CAS number 42399-41-7), gentamicin (CAS number 1403-66-3), neomycin (CAS number 1404-04-2), mecamylamine (CAS number 60-40-2), streptomycin (CAS number 57-92-1),
- modulators of **platelets aggregation** preferably selected from cilostazol (CAS number 73963-72-1), tirofiban (CAS number 144494-65-5), clopidogrel (CAS number 113665-84-2), ticlopidine (CAS number 55142-85-3).

Therapy according to the invention may be performed as drug combination and/or in conjunction with any other therapy. It may be provided at home, the doctor's office, a clinic, a hospital's outpatient department, or a hospital, so that the doctor can observe the therapy's effects closely and make any adjustments that are needed.

The duration of the therapy depends on the stage of the disease being treated, the age and condition of the patient, and how the patient responds to the treatment.

The dosage, frequency and mode of administration of each component of the combination can be controlled independently. For example, one drug may be administered orally while the second drug may be administered intramuscularly. Combination therapy may be given in on-and-off cycles that include rest periods so that the patient's body has a chance to recovery from any as yet unforeseen side-effects. The drugs may also be formulated together such that one administration delivers both drugs.

### Formulation of Pharmaceutical Compositions

The administration of each drug of the combination may be by any suitable means that results in a concentration of the drug that, combined with the other component, is able to diminish neuroinflammation.

While it is possible for the active ingredients of the combination to be administered as the pure chemical, it is preferable to present them as a pharmaceutical composition, also referred to in this context as pharmaceutical formulation. Possible compositions include those suitable for oral, rectal, topical (including transdermal, buccal and sublingual), or parenteral (including subcutaneous, intramuscular, intravenous and intradermal) administration.

More commonly these pharmaceutical formulations are prescribed to the patient in "patient packs" containing a number dosing units or other means for administration of metered unit doses for use during a distinct treatment period in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in traditional prescriptions. The inclusion of a package insert has been shown to improve patient compliance with the physician's instructions. Thus, the invention further includes a pharmaceutical formulation, as herein before described, in combination with packaging material suitable for said formulations. In such a patient pack the intended use of a formulation for the combination treatment can be inferred by instructions, facilities, provisions, adaptations and/or other means to help using the formulation most suitably for the treatment. Such measures make a patient pack specifically suitable for and adapted for use for treatment with the combination of the present invention.

The drug may be contained in any appropriate amount in any suitable carrier substance, and may be present in an amount of 1-99% by weight of the total weight of the composition. The composition may be provided in a dosage form that is suitable for the oral, parenteral (e.g., intravenously, intramuscularly), rectal, cutaneous, nasal, vaginal, inhalant, skin (patch), or ocular administration route. Thus, the composition may be in the form of, e.g., tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, or aerosols.

The pharmaceutical compositions may be formulated according to conventional pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Pharmaceutical compositions according to the invention may be formulated to release the active drug substantially immediately upon administration or at any predetermined time or time period after administration.

The controlled release formulations include (i) formulations that create a substantially constant concentration of the drug within the body over an extended period of time; (ii) formulations that after a predetermined lag time create a substantially constant concentration of the drug within the body over an extended period of time; (iii) formulations that sustain drug action during a predetermined time period by maintaining a relatively, constant, effective drug level in the body with concomitant minimization of undesirable side effects associated with fluctuations in the plasma level of the active drug substance; (iv) formulations that localize drug action by, e.g., spatial placement of a controlled release regimen adjacent to or in the diseased tissue or organ; and (v) formulations that target drug action by using carriers or chemical derivatives to deliver the drug to a particular target cell type.

Administration of drugs in the form of a controlled release formulation is especially preferred in cases in which the drug, either alone or in combination, has (i) a narrow therapeutic index (i.e., the difference between the plasma concentration leading to harmful side effects or toxic reactions and the plasma concentration leading to a therapeutic effect is small; in general, the therapeutic index, TI, is defined as the ratio of median lethal dose (LD50) to median effective dose (ED50)); (ii) a narrow absorption window in the gastro-intestinal tract; or (iii) a very short biological half-life so that frequent dosing during a day is required in order to sustain the plasma level at a therapeutic level.

Any of a number of strategies can be pursued in order to obtain controlled release in which the rate of release outweighs the rate of metabolism of the drug in question. Controlled release may be obtained by appropriate selection of various formulation parameters and ingredients, including, e.g., various types of controlled release compositions and coatings. Thus, the drug is formulated with appropriate excipients into a pharmaceutical regimen that, upon administration, releases the drug in a controlled manner (single or multiple unit tablet or capsule compositions, oil solutions, suspensions, emulsions, microcapsules, microspheres, nanoparticles, patches, and liposomes).

### Solid Dosage Forms for Oral Use

Formulations for oral use include tablets containing the active ingredient(s) in a mixture with non-toxic pharmaceutically acceptable excipients. These excipients may be, for example, inert diluents or fillers (e.g., sucrose, microcrystalline cellulose, starches including potato starch, calcium carbonate, sodium chloride, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., acacia, alginic acid, sodium alginate, gelatin, starch, pregelatinized starch, microcrystalline cellulose, carboxymethylcellulose sodium, methylcellulose, hydroxypropyl methylcellulose, ethylcellulose, polyvinylpyrrolidone, or polyethylene glycol); and lubricating agents, glidants, and antiadhesives (e.g., stearic acid, silicas, or talc). Other pharmaceutically acceptable excipients can be colorants, flavoring agents, plasticizers, humectants, buffering agents, and the like.

The tablets may be uncoated or they may be coated by known techniques, optionally to delay disintegration and absorption in the gastrointestinal tract and thereby providing a sustained action over a longer period. The coating may be adapted to release the active drug substance in a predetermined pattern (e.g., in order to achieve a controlled release formulation) or it may be adapted not to release the active drug substance until after passage of the stomach (enteric coating). The coating may be a sugar coating, a film coating (e.g., based on hydroxypropyl methylcellulose, methylcellulose, methyl hydroxyethylcellulose, hydroxypropylcellulose, carboxymethylcellulose, acrylate copolymers, polyethylene glycols and/or polyvinylpyrrolidone), or an enteric coating (e.g., based on methacrylic acid copolymer, cellulose acetate phthalate, hydroxypropyl methylcellulose phthalate, hydroxypropyl methylcellulose acetate succinate, polyvinyl acetate phthalate, shellac, and/or ethylcellulose). A time delay material such as, e.g., glyceryl monostearate or glyceryl distearate may be employed.

The solid tablet compositions may include a coating adapted to protect the composition from unwanted chemical changes, (e.g., chemical degradation prior to the release of the active drug substance). The coating may be applied on the solid dosage form in a similar manner as that described in Encyclopedia of Pharmaceutical Technology.

The two compounds may be mixed together in the tablet, or may be partitioned. For example, the first compound is contained on the inside of the tablet, and the second compound is on the outside, such that a substantial portion of the second compound is released prior to the release of the first compound.

Formulations for oral use may also be presented as chewable tablets, or as hard gelatin capsules wherein the active ingredient is mixed with an inert solid diluent (e.g., potato starch, microcrystalline cellulose, calcium carbonate, calcium phosphate or kaolin), or as soft gelatin capsules wherein the active ingredient is mixed with water or an oil medium, for example, liquid paraffin, or olive oil. Powders and granulates may be prepared using the ingredients mentioned above under tablets and capsules in a conventional manner.

Controlled release compositions for oral use may, e.g., be constructed to release the active drug by controlling the dissolution and/or the diffusion of the active drug substance.

Dissolution or diffusion controlled release can be achieved by appropriate coating of a tablet, capsule, pellet, or granulate formulation of drugs, or by incorporating the drug into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-hydroxymethacrylate, methacrylate hydrogels, 1,3 butylene glycol, ethylene glycol methacrylate, and/or polyethylene glycols. In a controlled release matrix formulation, the matrix material may also include, e.g., hydrated metylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

A controlled release composition containing one or more of the compounds of the claimed combinations may also be in the form of a buoyant tablet or capsule (i.e., a tablet or capsule that, upon oral administration, floats on top of the gastric content for a certain period of time). A buoyant tablet formulation of the drug(s) can be prepared by granulating a mixture of the drug(s) with excipients and 20-75% w/w of hydrocolloids, such as hydroxyethylcellulose, hydroxypropylcellulose, or hydroxypropylmethylcellulose. The obtained granules can then be compressed into tablets. On contact with the gastric juice, the tablet forms a substantially water-impermeable gel barrier around its surface. This gel barrier takes part in maintaining a density of less than one, thereby allowing the tablet to remain buoyant in the gastric juice.

### Liquids for Oral Administration

Powders, dispersible powders, or granules suitable for preparation of an aqueous suspension by addition of water are convenient dosage forms for oral administration. Formulation as a suspension provides the active ingredient in a mixture with a dispersing or wetting agent, suspending agent, and one or more preservatives. Suitable suspending agents are, for example, sodium carboxymethylcellulose, methylcellulose, sodium alginate, and the like.

### Parenteral Compositions

The pharmaceutical composition may also be administered parenterally by injection, infusion or implantation (intravenous, intramuscular, subcutaneous, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation.

Compositions for parenteral use may be provided in unit dosage forms (e.g., in single-dose ampoules), or in vials containing several doses and in which a suitable preservative may be added (see below). The composition may be in form of a solution, a suspension, an emulsion, an infusion device, or a delivery device for implantation or it may be presented as a dry powder to be reconstituted with water or another suitable vehicle before use. Apart from the active drug(s), the composition may include one or more suitable parenterally acceptable carriers and/or excipients. The active drug(s) may be incorporated into microspheres, microcapsules, nanoparticles, liposomes, or the like for controlled release. The composition may include suspending, solubilizing, stabilizing, pH-adjusting agents, and/or dispersing agents.

The pharmaceutical compositions according to the invention may be in the form suitable for sterile injection. To prepare such a composition, the suitable active drug(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution. The aqueous formulation may also contain one or more preservatives (e.g., methyl, ethyl or n-propyl p-hydroxybenzoate). In cases where one of the drugs is only sparingly or slightly soluble in water, a dissolution enhancing or solubilizing agent can be added, or the solvent may include 10-60% w/w of propylene glycol or the like.

Controlled release parenteral compositions may be in form of aqueous suspensions, microspheres, microcapsules, magnetic microspheres, oil solutions, oil suspensions, or emulsions. Alternatively, the active drug(s) may be incorporated in biocompatible carriers, liposomes, nanoparticles, implants, or infusion devices. Materials for use in the preparation of microspheres and/or microcapsules are, e.g., biodegradable/bioerodible polymers such as polygalactin, poly-(isobutyl cyanoacrylate), poly(2-hydroxyethyl-L-glutamnine). Biocompatible carriers that may be used when formulating a controlled release parenteral formulation are carbohydrates (e.g., dextrans), proteins (e.g., albumin), lipoproteins, or antibodies. Materials for use in implants can be non-biodegradable (e.g., polydimethyl siloxane) or biodegradable (e.g., poly(caprolactone), poly(glycolic acid) or poly(ortho esters).

### Rectal Compositions

For rectal application, suitable dosage forms for a composition include suppositories (emulsion or suspension type), and rectal gelatin capsules (solutions or suspensions). In a typical suppository formulation, the active drug(s) are combined with an appropriate pharmaceutically acceptable suppository base such as cocoa butter, esterified fatty acids, glycerinated gelatin, and various water-soluble or dispersible bases like polyethylene glycols. Various additives, enhancers, or surfactants may be incorporated.

### Percutaneous and Topical Compositions

The pharmaceutical compositions may also be administered topically on the skin for percutaneous absorption in dosage forms or formulations containing conventionally non-toxic pharmaceutical acceptable carriers and excipients including microspheres and liposomes. The formulations include creams, ointments, lotions, liniments, gels, hydrogels, solutions, suspensions, sticks, sprays, pastes, plasters, and other kinds of transdermal drug delivery systems. The pharmaceutically acceptable carriers or excipients may include emulsifying agents, antioxidants, buffering agents, preservatives, humectants, penetration enhancers, chelating agents, gel-forming agents, ointment bases, perfumes, and skin protective agents.

### The emulsifying agents may be naturally occurring gums (e.g., gum acacia or gum tragacanth)

The preservatives, humectants, penetration enhancers may be parabens, such as methyl or propyl p-hydroxybenzoate, and benzalkonium chloride, glycerin, propylene glycol, urea, etc.

The pharmaceutical compositions described above for topical administration on the skin may also be used in connection with topical administration onto or close to the part of the body that is to be treated. The compositions may be adapted for direct application or for application by means of special drug delivery devices such as dressings or alternatively plasters, pads, sponges, strips, or other forms of suitable flexible material.

### Dosages and duration of the treatment

It will be appreciated that the drugs of the combination may be administered concomitantly, either in the same or different pharmaceutical formulation or sequentially. If there is sequential administration, the delay in administering the second (or additional) active ingredient should not be such as to lose the benefit of the efficacious effect of the combination of the active ingredients. A minimum requirement for a combination according to this description is that the combination should be intended for combined use with the benefit of the efficacious effect of the combination of the active ingredients. The intended use of a combination can be inferred by facilities, provisions, adaptations and/or other means to help using the combination according to the invention.

Therapeutically effective amounts of two or more drugs that are subjects of this invention can be used together for the preparation of a medicament useful for reducing neuroinflammation once it has become clinically manifest or to prevent this condition for patients at risk.

Although the active drugs of the present invention may be administered in divided doses, for example two or three times daily, a single daily dose of each drug in the combination is preferred, with a single daily dose of all drugs in a single pharmaceutical composition (unit dosage form) being most preferred.

Administration can be one to several times daily for several days to several years, and may even be for the life of the patient. Chronic or at least periodically repeated long-term administration will be indicated in most cases.

The term "unit dosage form" refers to physically discrete units (such as capsules, tablets, or loaded syringe cylinders) suitable as unitary dosages for human subjects, each unit containing a predetermined quantity of active material or materials calculated to produce the desired therapeutic effect, in association with the required pharmaceutical carrier.

The amount of each drug in the combination preferred for a unit dosage will depend upon several factors including the administration method, the body weight and the age of the patient, the severity of neuroiflammation process or risk of potential side effects considering the general health status of the person to be treated.

Additionally, pharmacogenomic (the effect of genotype on the pharmacokinetic, pharmacodynamic or efficacy profile of a therapeutic) information about a particular patient may affect the dosage used.

Except when responding to especially impairing neuroinflammation when higher dosages may be required, or when treating children when lower dosages should be chosen, the preferred dosage of each drug in the combination will usually lie within the range of doses not above the usually prescribed for long-term maintenance treatment or proven to be safe in the large phase 3 clinical studies.

It will be understood that the amount of the drug actually administered will be determined by a physician, in the light of the relevant circumstances including the condition or conditions to be treated, the exact regimen to be administered, the age, weight, and response of the individual patient, the severity of the patient's symptoms, and the chosen route of administration. Therefore, the above dosage ranges are intended to provide general guidance and support for the teachings herein, but are not intended to limit the scope of the invention.

The following examples are given for purposes of illustration and not by way of limitation.

### EXAMPLES

### Materials and Methods

### Protocol for the Induction of EAE in C57BL/6 Mice

A model in which myelin-oligodendrocyte glycoprotein-immunized (MOG-immunized) mice develop chronic progressive EAE is used to demonstrate the beneficial effect of compositions of the invention in neuroinflamation treatment.

### I- Animals and Chemicals

SJL and C57BL/6J mice are purchased from Janvier (France). C57BL/6 mice, female of 6- to 9-week-old (Optimal: 7-week-old); Complete Freund's adjuvant (CFA): mixing 20 mL IFA (Adjuvant Incomplete Freund, DIFCO 263910) with 100 mg M.tuberculosis H37 Ra (killed and desiccated, DIFCO 231141); 3mM MOG35-55 peptide (MEVGWYRSPFSRVVHLYRNGK) stock dissolved in PBS.

### II- Procedure

The experimental encephalomyelitis is induced by following procedure:
Day 0:
   Prepare the emulsion containing MOG35-55 stock, PBS and CFA. The volume of MOG35-55 stock = [(The volume of the emulsion) × (1.5 g/L)] / [(3×10-3 mol/L) × (2581.6 g/mol)]; the volume of PBS = [(The volume of the emulsion) / 2] - (The volume of MOG35-55 stock); The volume of CFA = (The volume of the emulsion) / 2.
   The emulsion is prepared by diluting the MOG35-55 stock with PBS and then mix equal volumes of this diluent and CFA. The final concentration of MOG35-55 is 300 µg per 200 µL emulsion for each mouse. Air bubbles should be avoided and the syringe must be kept at 4°C until injection. Before use the stability of the emulsion is tested by adding one drop into a beaker of water: if the drop remains as a solid clump and slowly dissipates, then the emulsion is stable and may be injected.
   Mice are anesthetized with 2% Pentobarbital sodium (i.p injection, 70~90 µL/mouse) and kept warm under infrared lamp. Injection of 200ng Pertussis toxin in 200 µL PBS/mouse is performed (i.v.; tail vein) using a 1-mL syringe with 0.4-mm needle. The previously described emulsion is subcutaneously administered with a 1-mL syringe with 0.4-mm needle.
Day 2:
   Mice are kept warm under infrared lamp to perform injection of 200 ng Pertussis toxin in 200 µL PBS via i.v. (tail vein) route, as described for MOG35-55 injection.
Days 0∼70:
   Mice are daily observed for clinical symptoms.

### III-Clinical score

0.0: No sign of disease
0.5: Partial paralysis of the tail: flaccidity and absence of curling in the distal half of the tail when mouse is picked up.
1.0: When mouse is picked up, on can observe a complete paralysis of the tail associated to flaccidity and absence of curling at the tip.
1.5: Weakness of one hind paw that does not allow mouse to hang on by one hind foot (the other hind foot is normal).When hanging on by the joint of the hind limb, this is not counted in the score.
2.0: Weakness of both hind paws: mouse cannot hang on by any of its hind feet. Mouse waddles along and has difficulty to raise its rump up completely with the hind paws.
2.5: Hind paw paralysis:

Partial paralysis of both hind paws resulting in the difficulty to move the paws.

Complete paralysis in one hind paw resulting in the impossibility to move it. By walking, this paw trails behind the mouse.

To allow mouse to feed, water and food are given on the cage floor.

3.0: Complete hind paw paralysis although fore-paws are not affected by pathology.

Mouse can still hang on thanks to its fore paws. To allow mouse to feed, water and food are given on the cage floor.

3.5: Partial fore-paw paralysis including a complete paralysis of one fore-paw or partial paralysis of both.

4.0: Complete fore-paws paralysis. Moribund state.

5.0: Death of animal or sacrifice for experimental ethics.

Animals are kept in a conventional pathogen-free facility and all experiments are carried out in accordance with guidelines prescribed by, and are approved by, the standing local committee of bioethics.

Statistics. Statistica software (Statsoft Inc.) is utilized throughout for statistical analysis.

ANOVA analysis and Student's t test are employed to analyze clinical disease score. P < 0.05 is considered significant.

### Acute drug treatment

Inhibition of Acute Onset EAE and Chronic EAE is assessed by tissue analysis (detection of the presence of perivascular lymphocyte infiltrate) as well as by clinical scoring and body mass weighting.

The acute onset study is performed for 28 days after EAE induction.

Females are divided into groups of 12 rats before EAE induction. The treatment is administered during 3 weeks before EAE induction and further throughout the whole experiment.

Clopidogrel and mecamylamine are administered alone or in combinations orally (clopidogrel) and subcutaneously (mecamylamine). Mecamylamine, at one of the doses ( 0,04 mg/kg 0,2 mg/kg, 1 mg/kg/day) , and a placebo control is delivered via subcutaneous osmotic minipump over 6 weeks. After 6 weeks, animals received anesthesia and a small incision is made in the skin after preparation for aseptic surgery.

The pump is then replaced with a new pump and drug or placebo is delivered over a next period of time.

Clopidogrel (dosed from 0,02 mg/kg/day to 1 mg/kg/day) is administered as clopidogrel tablets dissolved in drinking water, Plavix, Bristol-Myers Squibb/Sanofi). The upper dose is known to be effective (De La CP et al. 2003) and safe based on results from experiments testing drug toxicity in rats (Reist M. et al. 2000).

All animals are daily examined for behavioural tests. The observations are performed by experimentator who does not know identity of treatments.

At the end of experiment, rats are intracardiacally perfused with saline. Spinal cord and brain tissues are removed and postfixed in formalin and then embedded in paraffin.

Sections of 2-4 micrometer are collected and stained with Hematoxylene and Eosin to be examined under light microscope for the presence of perivascular lymphocyte infiltrate inflammation. Scores are then attributed as no inflammation, mild, moderate and severe as described (Mohamed, 2004).

### The interpretation of results

1 Histological examination at day 28 reveals that majority of animals in the group treated with the combination of clopidogrel and mecamylamine have no inflammation (no signs of perivascular cuffing), with the rest of the animals showed only mild perivascular cuffing in the spinal section, while the animals from the placebo group and from the groups treated with clopidogrel or mecamylamine alone showed different degrees of perivascular inflammation.
   This difference between the group treated with the combination of two drugs and the control placebo groups reaches statistical significance (p value less then 0.05).
2 In comparison to the mice receiving vehicle, the mice receiving the combination of clopidogrel and mecamylamine exhibit a significantly lower mean clinical score (p value less then 0.05) at day 28 of the experiment then mice from all other experimental groups of animals induced fro EAE. These results demonstrate that this combination decreases both the severity of EAE, as compared to control placebo group.

### Chronic drug treatment.

A chronic phase study is run for 70 days post EAE induction.

All treated animals are receiving the treatment during three weeks prior to EAE induction. The combined clopidogrel and mecamylamine are administered alone or in combinations orally (clopidogrel) and subcutaneously (mecamylamine).

Mecamylamine, at one of three doses (0,05 mg/kg 0,2 mg/kg, 1 mg/kg/day) , and a placebo control is delivered via subcutaneous osmotic minipump over 6 weeks. After 6 weeks, animals received anesthesia and a small incision is made in the skin after preparation for aseptic surgery. The pump is then replaced with a new pump and drug or placebo is delivered over a next period of time.

Clopidogrel (20 mg/kg/day) is administered as clopidogrel tablets dissolved in drinking water, Plavix, Bristol-Myers Squibb/Sanofi).

For the study female animals are divided into groups of 12 rats each and subjected to EAE induction.

All animals are examined for behavioural deficits daily; the examinations are done by an experimentator who is blinded as to the treatments they received. All control animals treated with placebo are symptomatic at 11-24 days.

### The interpretation of results

In comparison to the mice receiving vehicle, the mice receiving the combination of clopidogrel and mecamylamine exhibit a significantly lower mean clinical score (p value less then 0.05) at day 70 of the experiment then mice from all other experimental groups of animals induced fro EAE. These results demonstrate that this combination decreases both the severity of EAE, as compared to control placebo group.

The differences in weight gain are correlated with the disease severity, i.e., stronger disease severity (higher mean clinical score) resulted in a greater weight loss. These differences are statistically significant (p<0.005, Manova). The subsequent Post Hoc comparison revealed that all the groups treated with clopidogrel or mecamylamine are different from the control group treated with placebo (vehicle) whereas the initial mean weights of all the groups are equal. On day 14, three days after the onset of the disease, sick mice lost an average of 2 g of weight. The average mean weight of mice treated with the combination of clopidogrel and mecamylamine is reduced by only 0.4 g. On day 28 the control group reached the lowest weight, 14.0 g.

The group treated with the combination of clopidogrel and mecamylamine is comparable with the positive control on day 28 (statistically there is no difference between these two groups) and significantly different from the sick mice treated with the placebo on day 70.

### REFERENCES

Adair-Kirk, TL et al. A site on laminin α5, AQARSAASKVKVSMKF, induces inflammatory cell production of matrix metalloproteinase-9 and chemotaxis. J. Immunol. 2003; 171: 398-406.
Adamson, P et al. Lymphocyte migration through brain endothelial cell monolayers involves signaling through endothelial ICAM-1 via a rho-dependent pathway. J. Immunol. 1999; 162: 2964-2973.
Agrawal SM & Yong VW. Immunopathogenesis of multiple sclerosis. Int Rev Neurobiol. 2007; 79: 99-126.
Allen IV & McKeown SR. A histological, histochemical and biochemical study of the macroscopically normal white matter in multiple sclerosis. J Neurol Sci. 1979; 41: 81-91.
Allen IV et al. Pathological abnormalities in the normal-appearing white matter in multiple sclerosis. Neurol Sci. 2001; 22: 141-4.
Alonso A & Heman MA. Temporal trends in the incidence of multiple sclerosis: a systematic review. Neurology. 2008; 71: 129-35.
Amit R et al. Acute severe combined demyelination. Childs Nerv Syst. 1992; 8: 354-6.
Amit R et al. Acute, severe, central and peripheral nervous system combined demyelination. Pediatr Neurol. 1986; 2: 47-50.
Anlar B et al. Acute disseminated encephalomyelitis in children: outcome and prognosis. Neuropediatrics. 2003; 34: 194-9.
Bergamaschi R et al. A case of relapsing neuromyelitis optica treated with glatiramer acetate. J Neurol. 2003; 250: 359-61.
Bevilacqua, MP et al. Recombinant tumor necrosis factor induces procoagulant activity in cultured human vascular endothelium: characterization and comparison with the actions of interleukin 1. Proc. Natl. Acad. Sci. USA 1986; 83: 4533-4537.
Birch, KA et al. Calcium/calmodulin transduces thrombin-stimulated secretion: studies in intact and minimally permeabilized human umbilical vein endothelial cells. J. Cell Biol. 1992; 118: 1501-1510.
Birch, KA et al. Prolonged peak elevations in cytoplasmic free calcium ions, derived from intracellular stores, correlate with the extent of thrombin-stimulated exocytosis in single human umbilical vein endothelial cells. J. Cell. Physiol. 1994; 160: 545-554.
Bjartmar C et al. Neurological disability correlates with spinal cord axonal loss and reduced N-acetyl aspartate in chronic multiple sclerosis patients. Ann Neurol. 2000; 48: 893-901.
Block ML & Hong JS. Microglia and inflammation-mediated neurodegeneration: multiple triggers with a common mechanism. Prog Neurobiol. 2005; 76: 77-98.
Butcher, EC. Leukocyte-endothelial cell recognition: three (or more) steps to specificity and diversity. Cell 1991; 67: 1033-1036.
Campbell, J.J et al. Chemokines and the arrest of lymphocytes rolling under flow conditions. Science 1998; 279: 381-384.
Campbell, JJ et al. Biology of chemokine and classical chemoattractant receptors. Differential requirements for adhesion-triggering versus chemotactic responses in lymphoid cells. J. Cell Biol. 1996; 134: 255-266.
Cardona AE et al. Chemokines in and out of the central nervous system: much more than chemotaxis and inflammation. J Leukoc Biol. 2008; 84: 587-94.
Carvey PM et al. 6-Hydroxydopamine-induced alterations in blood-brain barrier permeability. Eur J Neurosci. 2005; 22: 1158-68.
Cree BA et al. An open label study of the effects of rituximab in neuromyelitis optica. Neurology. 2005; 64: 1270-2.
Cree BA et al. Clinical characteristics of African Americans vs Caucasian Americans with multiple sclerosis. Neurology. 2004; 63: 2039-45.
Cree BA et al. Neuromyelitis optica. Semin Neurol. 2002; 22: 105-22.
Dangerfield, J et al. PECAM-1 (CD31) homophilic interaction up-regulates α6β1 on transmigrated neutrophils in vivo and plays a functional role in the ability of α6 integrins to mediate leukocyte migration through the perivascular basement membrane. J. Exp. Med. 2002; 196: 1201-1211.
De La CP et al. Effects of clopidogrel and ticlopidine on experimental diabetic ischemic retinopathy in rats. Naunyn Schmiedebergs Arch Pharmacol. 2003; 367: 204-210. DeLuca GC et al. Axonal loss in multiple sclerosis: a pathological survey of the corticospinal and sensory tracts. Brain. 2004; 127: 1009-18.
Durieu-Trautmann, O et al. Intercellular adhesion molecule 1 activation induces tyrosine phosphorylation of the cytoskeleton-associated protein cortactin in brain microvessel endothelial cells. J. Biol. Chem. 1994; 269: 12536-12540.
Engelhardt, B & Wolburg, H Mini-review: transendothelial migration of leukocytes: through the front door or around the side of the house? Eur. J. Immunol. 2004; 34: 2955-2963.
Etienne, S et al. ICAM-1 signaling pathways associated with Rho activation in microvascular brain endothelial cells. J. Immunol. 1998; 161:5755-5761.
Ferguson B et al. Axonal damage in acute multiple sclerosis lesions. Brain. 1997; 120 ( Pt 3): 393-9.
Flugel A et al. Migratory activity and functional changes of green fluorescent effector cells before and during experimental autoimmune encephalomyelitis. Immunity. 2001; 14: 547-60.
Ganter P et al. Spinal cord axonal loss in multiple sclerosis: a post-mortem study. Neuropathol Appl Neurobiol. 1999; 25: 459-67.
Gartzen K et al. Relapsing neuromyelitis optica responsive to glatiramer acetate treatment. Eur J Neurol. 2007; 14: e12-3.
Ghezzi A et al. Clinical characteristics, course and prognosis of relapsing Devic's Neuromyelitis Optica. J Neurol. 2004; 251: 47-52.
Ghosh N et al. Evidence of axonal damage in human acute demyelinating diseases. J Neurol Sci. 2004; 222: 29-34.
Gonzalez-Scarano F & Baltuch G. Microglia as mediators of inflammatory and degenerative diseases. Annu Rev Neurosci. 1999; 22: 219-40.
Greenwood, J. et al. Intracellular domain of brain endothelial intercellular adhesion molecule-1 is essential for T lymphocyte-mediated signaling and migration. J. Immunol. 2003; 171: 2099-2108.
Haines JL et al. Linkage of the MHC to familial multiple sclerosis suggests genetic heterogeneity. The Multiple Sclerosis Genetics Group. Hum Mol Genet. 1998; 7: 1229-34.
Hayes CE. Vitamin D: a natural inhibitor of multiple sclerosis. Proc Nutr Soc. 2000; 59: 531-5.
Hickey WF et al. T-lymphocyte entry into the central nervous system. J Neurosci Res. 1991; 28: 254-60.
Hochmeister S et al. Dysferlin is a new marker for leaky brain blood vessels in multiple sclerosis. J Neuropathol Exp Neurol. 2006; 65: 855-65.
Hynson JL et al. Clinical and neuroradiologic features of acute disseminated encephalomyelitis in children. Neurology. 2001; 56: 1308-12.
Idrissova Zh R et al. Acute disseminated encephalomyelitis in children: clinical features and HLA-DR linkage. Eur J Neurol. 2003; 10: 537-46.
Ishii T & Haga S. Immuno-electron microscopic localization of immunoglobulins in amyloid fibrils of senile plaques. Acta Neuropathol. 1976; 36: 243-9.
Itagaki S et al. Presence of T-cytotoxic suppressor and leucocyte common antigen positive cells in Alzheimer's disease brain tissue. Neurosci Lett. 1988; 91: 259-64.
Jordan, SP & Sessa, PWC Evolving functions of endothelial cells in inflammation. Nature Reviews Immunology 2007; 7:803-815.
Kampman MT & Brustad M. Vitamin D: a candidate for the environmental effect in multiple sclerosis - observations from Norway. Neuroepidemiology. 2008; 30: 140-6.
Keegan M et al. Plasma exchange for severe attacks of CNS demyelination: predictors ofresponse. Neurology. 2002; 58: 143-6.
Kim SU & de Vellis. Microglia in health and disease. J Neurosci Res. 2005; 81: 302-13.
Kirk J et al. Tight junctional abnormality in multiple sclerosis white matter affects all calibres of vessel and is associated with blood-brain barrier leakage and active demyelination. J Pathol. 2003; 201: 319-27.
Konstantinopoulos PA et al. Selective modulation of the erythropoietic and tissue-protective effects of erythropoietin: time to reach the full therapeutic potential of erythropoietin. Biochim Biophys Acta. 2007; 1776: 1-9.
Kortekaas R et al. Blood-brain barrier dysfunction in parkinsonian midbrain in vivo. Ann Neurol. 2005; 57: 176-9.
Kreutzberg GW Microglia: a sensor for pathological events in the CNS. Trends Neurosci. 1996; 19: 312-8.
Kutzelnigg A et al. Cortical demyelination and diffuse white matter injury in multiple sclerosis. Brain. 2005; 128: 2705-12.
Kvietys, PR & Sandig, M Neutrophil diapedesis: paracellular or transcellular. News Physiol. Sci. 2001; 16:15-19.
Lassmann H et al. The immunopathology of multiple sclerosis: an overview. Brain Pathol. 2007; 17: 210-8.
Lassmann H. Models of multiple sclerosis: new insights into pathophysiology and repair. Curr Opin Neurol. 2008; 21: 242-7.
Leake JA et al. Acute disseminated encephalomyelitis in childhood: epidemiologic, clinical and laboratory features. Pediatr Infect Dis J. 2004; 23: 756-64.
Lehmann HC et al. Plasma exchange in neuroimmunological disorders: Part 1: Rationale and treatment of inflammatory central nervous system disorders. Arch Neurol. 2006; 63: 930-5.
Ley, K et al. Getting to the site of inflammation: the leukocyte adhesion cascade updated. Nature Reviews Immunology 2007; 7:678-689.
Li W et al. Beneficial effect of erythropoietin on experimental allergic encephalomyelitis. Ann Neurol. 2004; 56: 767-77.
Licandro A et al. Alzheimer's disease and senile brains: an immunofluorescence study. Riv Patol Nerv Ment. 1983; 104: 75-87.
Lincoln JA et al. Could Epstein-Barr virus or canine distemper virus cause multiple sclerosis? Neurol Clin. 2008; 26: 699-715, viii.
Lossinsky AS & Shivers RR. Structural pathways for macromolecular and cellular transport across the blood-brain barrier during inflammatory conditions. Review. Histol Histopathol. 2004; 19: 535-64.
Lovas G et al. Axonal changes in chronic demyelinated cervical spinal cord plaques. Brain. 2000; 123 ( Pt 2): 308-17.
Lucchinetti CF et al. A role for humoral mechanisms in the pathogenesis of Devic's neuromyelitis optica. Brain. 2002; 125: 1450-61.
Man S et al. Inflammatory cell migration into the central nervous system: a few new twists on an old tale. Brain Pathol. 2007; 17: 243-50.
Mandler RN et al. Devic's neuromyelitis optica: a prospective study of seven patients treated with prednisone and azathioprine. Neurology. 1998; 51: 1219-20.
Mann DM et al. Immunohistochemical staining of senile plaques. Neuropathol Appl Neurobiol. 1982; 8: 55-61.
Marchioni E et al. Postinfectious inflammatory disorders: subgroups based on prospective follow-up. Neurology. 2005; 65: 1057-65.
Mattiace LA et al. Detection of HLA-DR on microglia in the human brain is a function of both clinical and technical factors. Am J Pathol. 1990; 136: 1101-14.
McFarland HF & Martin R. Multiple sclerosis: a complicated picture of autoimmunity. Nat Immunol. 2007; 8: 913-9.
McGeer PL et al. Immune system response in Alzheimer's disease. Can J Neurol Sci. 1989; 16: 516-27.
Menge T et al. Acute disseminated encephalomyelitis: an acute hit against the brain. Curr Opin Neurol. 2007; 20: 247-54.
Millan, J & Ridley, AJ Rho GTPases and leucocyte-induced endothelial remodelling. Biochem. J. 2005; 385:329-337.
Miller DH et al. Measurement of atrophy in multiple sclerosis: pathological basis, methodological aspects and clinical relevance. Brain. 2002; 125: 1676-95.
Mohamed A et al. The use of digital technology to asses the severity of the Experimental Allergic Encephalomyelitis (EAE) spinal cord lesion. Biomed Sci Instrum. 2004; 40:419-23.
Muller, WA. Leukocyte-endothelial-cell interactions in leukocyte transmigration and the inflammatory response. Trends Immunol. 2003; 24:326-333.
Muller, WA. Migration of leukocytes across endothelial junctions: some concepts and controversies. Microcirculation 2001; 8:181-193.
Nadkarni N & Lisak RP. Guillain-Barre syndrome (GBS) with bilateral optic neuritis and central white matter disease. Neurology. 1993; 43: 842-3.
Nawroth, R et al. VE-PTP and VE-cadherin ectodomains interact to facilitate regulation of phosphorylation and cell contacts. EMBO J. 2002; 21:4885-4895.
Neuhaus O et al. Mechanisms of action of glatiramer acetate in multiple sclerosis. Neurology. 2001; 56: 702-8.
Neuhaus O et al. Putative mechanisms of action of statins in multiple sclerosiscomparison to interferon-beta and glatiramer acetate. J Neurol Sci. 2005; 233: 173-7.
Neumann H. Control of glial immune function by neurons. Glia. 2001; 36: 191-9.
Neumann H & Wekerle H. Neuronal control of the immune response in the central nervous system: linking brain immunity to neurodegeneration. J Neuropathol Exp Neurol. 1998; 57: 1-9.
Newman, PJ & Newman DK Signal transduction pathways mediated by PECAN-1: new roles for an old molecule in platelet and vascular cell biology. Arterioscler. Thromb. Vasc. Biol. 2003; 23: 953-964.
Nielsen S et al. Specialized membrane domains for water transport in glial cells: high-resolution immunogold cytochemistry of aquaporin-4 in rat brain. J Neurosci. 1997; 17: 171-80.
O'Connor PW et al. Randomized multicenter trial of natalizumab in acute MS relapses: clinical and MRI effects. Neurology. 2004; 62: 2038-43.
Oh HH et al. Molecular analysis of HLA class II-associated susceptibility to neuroinflammatory diseases in Korean children. J Korean Med Sci. 2004; 19: 426-30.
Olsson T & Hillert J. The genetics of multiple sclerosis and its experimental models. Curr Opin Neurol. 2008; 21: 255-60.
Pan, J et al. Comparison of promoters for the murine and human P-selectin genes suggests species-specific and conserved mechanisms for transcriptional regulation in endothelial cells. J. Biol. Chem. 1998; 273: 10058-10067.
Papeix C et al. Immunosuppressive therapy is more effective than interferon in neuromyelitis optica. Mult Scler. 2007; 13: 256-9.
Perlmutter LS et al. MHC class II-positive microglia in human brain: association with Alzheimer lesions. J Neurosci Res. 1992; 33: 549-58.
Pittock SJ et al. Brain abnormalities in neuromyelitis optica. Arch Neurol. 2006a; 63: 390-6.
Pittock SJ et al. Neuromyelitis optica brain lesions localized at sites of high aquaporin 4 expression. Arch Neurol. 2006b; 63: 964-8.
Polman CH et al. A randomized, placebo-controlled trial of natalizumab for relapsing multiple sclerosis. N Engl J Med. 2006; 354: 899-910.
Ponsonby AL et al. UVR, vitamin D and three autoimmune diseases--multiple sclerosis, type 1 diabetes, rheumatoid arthritis. Photochem Photobiol. 2005; 81: 1267-75.
Poser CM. Multiple sclerosis and recurrent disseminated encephalomyelitis are different diseases. Arch Neurol. 2008; 65: 674; author reply 674-5.
Posnett DN. Herpesviruses and autoimmunity. Curr Opin Investig Drugs. 2008; 9: 505-14.
Prat A et al. Glial cell influence on the human blood-brain barrier. Glia. 2001; 36: 145-55.
Prineas JW et al. Immunopathology of secondary-progressive multiple sclerosis. Ann Neurol. 2001; 50: 646-57.
Pugliatti M et al. Environmental risk factors in multiple sclerosis. Acta Neurol Scand Suppl. 2008; 188: 34-40.
Reist M, et al. Very slow chiral inversion of clopidogrel in rats: a pharmacokinetic and mechanistic investigation. Drug Metab Dispos. 2000; 28: 1405-1410.
Rogers J et al. Expression of immune system-associated antigens by cells of the human central nervous system: relationship to the pathology of Alzheimer's disease. Neurobiol Aging. 1988; 9: 339-49.
Rosati G. The prevalence of multiple sclerosis in the world: an update. Neurol Sci. 2001; 22: 117-39.
Rubin LL & Staddon JM. The cell biology of the blood-brain barrier. Annu Rev Neurosci. 1999; 22: 11-28.
Rust RS. Multiple sclerosis, acute disseminated encephalomyelitis, and related conditions. Semin Pediatr Neurol. 2000; 7: 66-90.
Saida T et al. Interferon beta-1b is effective in Japanese RRMS patients: a randomized, multicenter study. Neurology. 2005; 64: 621-30.
Schenkel, AR et al. Locomotion of monocytes on endothelium is a critical step during extravasation. Nature Immunol. 2004a; 5:393-400.
Schenkel, AR et al. Platelet endothelial cell adhesion molecule deficiency or blockade significantly reduces leukocyte emigration in a majority of mouse strains. J Immunol 2004b; 173:6403-6408.
Singh VK. Neuroautoimmunity: pathogenic implications for Alzheimer's disease. Gerontology. 1997; 43: 79-94.
Skaper SD. The brain as a target for inflammatory processes and neuroprotective strategies. Ann N Y Acad Sci. 2007; 1122: 23-34.
Sohn, RH et al. Regulation of endothelial thrombomodulin expression by inflammatory cytokines is mediated by activation of nuclear factor- kappa B. Blood 2005; 105:3910-3917.
Sotelo J. On the viral hypothesis of multiple sclerosis: participation of varicella-zoster virus. J Neurol Sci. 2007; 262: 113-6.
Springer, TA. Traffic signals for lymphocyte recirculation and leukocyte emigration: the multistep paradigm. Cell 1994; 76: 301-314.
Stadelmann C et al. Cortical pathology in multiple sclerosis. Curr Opin Neurol. 2008; 21: 229-34.
Stoll G & Jander S. The role of microglia and macrophages in the pathophysiology of the CNS. Prog Neurobiol. 1999; 58: 233-47.
Szpak GM et al. Neurones and microglia in central nervous system immune response to degenerative processes. Part 1: Alzheimer's disease and Lewy body variant of Alzheimer's disease. Quantitative study. Folia Neuropathol. 2001; 39: 181-92.
Tenembaum S et al. Acute disseminated encephalomyelitis. Neurology. 2007; 68: S23-36.
Tenembaum S et al. Acute disseminated encephalomyelitis: a long-term follow-up study of 84 pediatric patients. Neurology. 2002; 59: 1224-31.
Tilghman, RW & Hoover, RL The Src-cortactin pathway is required for clustering of E-selectin and ICAM-1 in endothelial cells. FASEB J. 2002; 16:1257-1259.
Tilleux S & Hermans E. Neuroinflammation and regulation of glial glutamate uptake in neurological disorders. J Neurosci Res. 2007; 85: 2059-70.
Trapp BD et al. Axonal transection in the lesions of multiple sclerosis. N Engl J Med. 1998; 338: 278-85.
Trapp BD & Nave KA. Multiple sclerosis: an immune or neurodegenerative disorder? Annu Rev Neurosci. 2008; 31: 247-69.
Trapp BD et al. Neurodegeneration in multiple sclerosis:relationship to neurological disability. Neuroscientist. 1999; 5: 48-57.
van Etten E & Mathieu C. Immunoregulation by 1,25-dihydroxyvitamin D3: basic concepts. J Steroid Biochem Mol Biol. 2005; 97: 93-101.
Vestweber, D. Adhesion and signaling molecules controlling the transmigration of leukocytes through endothelium. Immunological Reviews 2007; 218:178-196.
Walker DG & Lue LF. Investigations with cultured human microglia on pathogenic mechanisms of Alzheimer's disease and other neurodegenerative diseases. J Neurosci Res. 2005; 81: 412-25.
Warabi Y et al. Interferon beta-1b exacerbates multiple sclerosis with severe optic nerve and spinal cord demyelination. J Neurol Sci. 2007; 252: 57-61.
Watanabe S et al. Low-dose corticosteroids reduce relapses in neuromyelitis optica: a retrospective analysis. Mult Scler. 2007; 13: 968-74.
Weinstock-Guttman B et al. Study of mitoxantrone for the treatment of recurrent neuromyelitis optica (Devic disease). Arch Neurol. 2006; 63: 957-63.
Wekerle H et al. Cellular immune reactivity within the CNS. Trends Neurosci. 1986; 9: 271-277.
Wingerchuk DM & Weinshenker BG. Neuromyelitis Optica. Curr Treat Options Neurol. 2005; 7: 173-182.
Wingerchuk DM & Weinshenker BG. Neuromyelitis optica: clinical predictors of a relapsing course and survival. Neurology. 2003; 60: 848-53.
Wingerchuk DM et al. The clinical course of neuromyelitis optica (Devic's syndrome). Neurology. 1999; 53: 1107-14.
Yednock TA et al. Prevention of experimental autoimmune encephalomyelitis by antibodies against alpha 4 beta 1 integrin. Nature. 1992; 356: 63-6.

## Claims

1. A composition comprising a combination of at least two compounds chosen from the group consisting of irbesartan, idraparinux, otamixaban, SR48692, mecamylamine, clopidogrel, or salts or prodrugs or derivatives or sustained release formulations thereof, for simultaneous, separate or sequential administration.

2. A composition comprising a combination of at least two compounds chosen from the group consisting of a modulator of endothelium permeability, a modulator of extracellular matrix formation, a modulator of cell adhesion and motility, a modulator of shear stress on endothelium, a modulator of platelets aggregation, or salts or prodrugs or derivatives or sustained release formulations thereof.

3. A composition comprising a combination of at least two compounds chosen from the group consisting of irbesartan, idraparinux, otamixaban, SR48692, mecamylamine, clopidogrel, or salts or prodrugs or derivatives or sustained release formulations thereof, for treating neuroinflammation, particularly associated with neurodegenerative, autoimmune, infectious, toxic or traumatic disorders.

4. A composition comprising a combination of at least two compounds chosen from the group consisting of a modulator of endothelium permeability, a modulator of extracellular matrix formation, a modulator of cell adhesion and motility, a modulator of platelets aggregation, or salts or prodrugs or derivatives or sustained release formulations thereof, for treating neuroinflammation, particularly associated with neurodegenerative, autoimmune, infectious, toxic or traumatic disorders.

5. The composition according to claim 3 or 4, wherein the neurodegenerative disorder is selected from the group consisting of multiple sclerosis (MS), Alzheimer's disease (AD), Parkinson's disease (PD), Amyotrophic lateral sclerosis (ALS), Acute disseminated encephalomyelitis (ADEM) and Neuromyelitis optica (NMO).

6. A composition comprising a combination of at least two compounds chosen from the group consisting of irbesartan, idraparinux, otamixaban, SR48692, clopidogrel, mecamylamine, or salts or prodrugs or derivatives or sustained release formulations thereof, for treating multiple sclerosis (MS).

7. The composition according to claim 2 or 4, wherein said composition comprises at least one modulator of extracellular matrix formation, preferably idraparinux or otamixaban, and at least one modulator of platelets aggregation, preferably clopidogrel, ticlopidine or tirofiban.

8. The composition according to claim 2 or 4, wherein said composition comprises at least one modulator of cell adhesion and motility, preferably terbinafine, and at least one modulator of platelets aggregation, preferably clopidogrel, ticlopidine or tirofiban.

9. The composition according to claim 2 or 4, wherein said composition comprises at least one modulator of extracellular matrix formation, preferably idraparinux or otamixaban, and at least one modulator of cell adhesion and motility, preferably tranexamic acid.

10. The composition according to claim 2 or 4, wherein said composition comprises at least one modulator of extracellular matrix formation, preferably idraparinux or otamixaban, and at least one modulator of cell adhesion and motility, preferably neurotensin receptor antagonist SR48692.

11. The composition according to claim 2 or 4, wherein said composition comprises at least one modulator of cell adhesion and motility, preferably bacitracin, and at least one modulator of platelets aggregation, preferably clopidogrel, ticlopidine or tirofiban.

12. The composition according to claim 2 or 4, wherein said composition comprises at least one modulator of cell adhesion and motility, preferably diosmin, and at least one modulator of platelets aggregation, preferably clopidogrel, ticlopidine or tirofiban.

13. The composition according to claim 2 or 4, wherein said composition comprises at least one modulator of endothelium permeability, preferably irbesartan, and at least one modulator of platelets aggregation, preferably clopidogrel, ticlopidine or tirofiban.

14. The composition according to claim 2 or 4, wherein said composition comprises at least one modulator of cell adhesion and motility, preferably tranexamic acid, and at least one modulator of platelets aggregation, preferably clopidogrel, ticlopidine or tirofiban.

15. The composition according to claim 2 or 4, wherein said composition comprises at least one modulator of cell adhesion and motility, preferably neurotensin receptor antagonist SR48692, and at least one modulator of platelets aggregation, preferably clopidogrel, ticlopidine or tirofiban.

16. The composition according to claim 2 or 4, wherein said composition comprises at least one modulator of extracellular matrix formation, preferably argatroban, and at least one modulator of cell adhesion and motility, preferably tranexamic acid.

17. The composition according to claim 2 or 4, wherein said composition comprises at least one modulator of shear stress on endothelium, preferably mecamylamine or neomycin, and at least one modulator of platelets aggregation, preferably clopidogrel, ticlopidine or tirofiban.

18. The composition according to claim 2 or 4, wherein said composition comprises at least a modulator of endothelium permeability, preferably irbesartan, a modulator of extracellular matrix formation, preferably idraparinux or otamixaban, a modulator of cell adhesion and motility, preferably SR48692, a modulator of shear stress on endothelium, preferably mecamylamine, and a modulator of platelets aggregation, preferably clopidogrel.

19. A composition of any one of preceding claims, wherein said compounds are administered simultaneously, separately or sequentially in a same subject.

20. A composition according to any one of preceding claims, wherein said compounds are formulated in solid or liquid dosage forms, with one or several pharmaceutically acceptable excipients, for single or repeated administration to a patient.

21. A therapeutic regimen comprising a combination of at least two compounds chosen from the group consisting of a modulator of endothelium permeability, a modulator of extracellular matrix formation, a modulator of cell adhesion and motility, a modulator of shear stress on endothelium, a modulator of platelets aggregation, or salts or prodrugs or derivatives or sustained release formulations thereof.

22. A method for treating neuroinflammation in a human or animal subject, comprising administering simultaneously, separately or sequentially by any oral, or parenteral or intrathecal or topical route or by inhalation or percutaneous or mucosal route to said subject, in need thereof, an effective amount of any combination of at least two compounds according to claims 1 to 20, or salts or prodrugs or derivatives or sustained release formulations thereof.

23. The method according to claim 22, comprising the following steps:
- assessing whether a subject has neuroinflammatory disease; and
- treating the subject having this disease with an effective amount of a composition according to any one of claims 1 to 20.

24. The method according to claim 23, wherein the step of assessing whether a subject has neuroinflammation is done by a DNA assay.

25. A method for assessing neuroinflammation treatment efficacy in a subject, wherein cells derived from the subject are exposed in vitro to a composition according to any one of claims 1 to 20.

26. A composition comprising a combination of at least mecamylamine and clopidogrel, or salts, prodrugs, derivatives or sustained release formulations thereof, for simultaneous, separate or sequential administration.
